# EUROPÄISCHE PATENTANMELDUNG

(11) **EP 3 473 212 A1**
(43) Veröffentlichungstag der Anmeldung: **24.04.2019**
(21) Anmeldenummer: 17197235.9
(22) Anmeldetag: 19.10.2017
(51) Int. Cl.: A61F 2/24, A61M 25/00

(54) **SYSTEM AUS KATHETER UND HERZKLAPPENPROTHESE**

(71) Anmelder: Biotronik AG, 8180 Bülach (CH)
(72) Erfinder: Keller, Mark, 5000 Aarau (CH)
(74) Vertreter: Galander, Marcus

(57) **Zusammenfassung**

Die Erfindung betrifft System aus einem Katheter (1) und einem Implantat, insbesondere einer Herzklappenprothese, welches ein Abknicken der Herzklappenprothese bei Resheathing verhindert. Die Herzklappenprothese besitzt eine selbstexpandierende Stützstruktur. Der Katheter umfasst einen Außenschaft (3) und einen in dem Außenschaft (3) angeordneten Innenschaft (7, 7a, 7b), wobei der Innenschaft (7, 7a, 7b) einen röhrenförmigen Abschnitt aufweist, welcher eine Vielzahl von hohlzylinderförmigen, in Längsrichtung nebeneinander angeordneten und zueinander verschiebbaren sowie zueinander schwenkbaren Segmenten (17, 17a, 17b) aufweist, wobei jedes Segment (17, 17a, 17b) in Längsrichtung an einem Ende eine erste Anzahl n (n ≥ 2) sich jeweils in Längsrichtung erstreckende Zapfen (18, 18a, 28, 30) und an dem anderen Ende jeweils eine der ersten Anzahl entsprechende Anzahl von Ausnehmungen (19, 19a, 29, 31) mit einer zu der Form des jeweiligen Zapfens (18, 18a, 28, 30) komplementären Form aufweist, wobei jeder Zapfen (18, 18a, 28, 30) des unmittelbar benachbarten Segments in die jeweilige Ausnehmung (19, 19a, 29, 31) mit der komplementären Form eingreift und umgekehrt, wobei eine zweite Anzahl m (m ≥ 1 und n ≥ m) der Zapfen (18, 18a, 28) des Segments (17, 17a, 17b) eine den Abstand in Längsrichtung der benachbarten Segmente (17, 17a, 17b) zueinander begrenzende Hinterschneidung (20, 20a, 20b) aufweist. Die Erfindung betrifft ferner ein entsprechendes röhrenförmiges Element für einen Innenschaft (7, 7a, 7b) eines Katheters und ein Verfahren zur Herstellung eines derartigen Systems.

## Beschreibung

Die vorliegende Erfindung betrifft ein System aus einem Katheter und einem Implantat mit einer selbstexpandierenden Stützstruktur. Die vorliegende Erfindung eignet sich besonders für ein Implantat, welches als Herzklappenprothese ausgebildet ist und eine Klappenanordnung umfasst, die an einem selbstexpandierenden Grundgerüst befestigt ist. Die Erfindung wird daher im Folgenden am Beispiel einer derartigen Herzklappenprothese beschrieben, ist aber nicht auf derartige Implantate beschränkt, sondern insbesondere auch für die im nächsten Abschnitt beschriebenen Implantate geeignet.

Medizinische Implantate und Prothesen, insbesondere intraluminale Endoprothesen, für unterschiedlichste Anwendungen sind in großer Vielfalt aus dem Stand der Technik bekannt. Prothesen sind z.B. Endoprothesen zum Verschließen von persistierenden Foramen Ovale (PFO), Endoprothesen zum Verschließen eines ASG (Vorhofscheidewanddefekt, Atrioseptaldefekt), Aortenprothesen sowie Prothesen im Bereich des Hart- und Weichgewebes oder selbstexpandierde Stents. Ein prominenter Vertreter der Prothesen sind Herzklappenprothesen, welche als künstlicher Ersatz für eine natürliche Herzklappe zur Verhinderung einer akuten oder chronischen Herzinsuffizienz verwendet werden. Herzklappenprothesen werden nach der offen-chirurgischen Technik oder in letzter Zeit zunehmend minimal-invasiv eingesetzt.

Für die Anwendung minimal-invasiver Verfahren im Bereich des Herzklappenersatzes ist heute insbesondere die Transkatheter-Aortenklappenimplantation (englisch: transcatheter aortic valve implantation TAVI) von Bedeutung. Bei dieser Implantationstechnik wird mittels eines Katheters eine Herzklappenprothese für die Aortenklappe transfemoral oder transkapital eingesetzt. Eine solche Herzklappenprothese besitzt häufig eine Stützstruktur sowie innerhalb der Stützstruktur angebrachte biegsame Klappensegel.

Aus der Druckschrift US 9,155,619 B2 sind eine Einrichtung zum Einbringen einer Herzklappenprothese und eine Herzklappenprothese bekannt. Die Einrichtung weist mindestens drei ineinander angeordnete Schäfte auf, wobei ein mittlerer Schaft als sogenannter Drehmomenten-Schaft ausgebildet ist, welcher eine Vielzahl von ringförmigen metallischen Gliedern aufweist. Diese sind über in proximale und distale Richtung von dem Glied abstehende Pins miteinander verbunden, sodass ein Drehmoment über die gesamte Länge des Schafts übertragen werden kann. Dieses System hat den Nachteil einer bevorzugten Biegerichtung, die nicht zwangsläufig mit der natürlichen Biegerichtung der Aorta des Patienten übereinstimmt und entsprechend auch die Aorta aus ihrer natürlichen Orientierung verschieben kann.

Eine Herzklappenprothese, welche kathetergestützt eingesetzt wird, nimmt üblicherweise zwei Zustände ein, nämlich einen komprimierten Zustand mit einem kleinen Durchmesser und einen expandierten Zustand mit einem größeren Durchmesser. Im komprimierten Zustand kann das Implantat mittels des Katheters durch enge Gefäße hindurch in das Herz eingeführt und im Bereich der Aortenklappe positioniert werden. Die Fixierung im komprimierten Zustand erfolgt hierbei häufig mittels einer am distalen Ende des Katheters angeordneten Katheterhülse (auch als Kapsel bezeichnet), die mit dem Außenschaft des Katheters verbunden ist. Die Herzklappenprothese ist dabei im distalen Bereich auf einem Innenschaft des Katheters aneordnet. Der Innenschaft schließt ein inneres Lumen ein, in dem ein Führungsdraht (guide wire) geführt wird. Der Innenschaft wird durch die Katheterhülse und den Außenschaft umgeben. Anders ausgedrückt ist der Innenschaft in dem Außenschaft und der Katheterhülse angeordnet. In dem expandierten Zustand ist die Herzklappenprothese vollständig entfaltet, und von dem Katheter abgetrennt und im Bereich der Aortenklappe verankert. Die körpereigene Aortenklappe wird dabei nicht entfernt, sondern durch das Implantat verdrängt. Anschließend wird der Katheter aus dem Körper des Behandelten entfernt. Die Expansion der Stützstruktur bewirkt die Entfaltung der Herzklappenprothese. Das Grundgerüst der Herzklappenprothese ist selbstexpandierend und beispielsweise aus einem Formgedächtnismaterial wie Nitinol ausgeführt. Der Außenschaft des Katheters und insbesondere sein distaler Endbereich, die Implantatkapsel, überdeckt die Herzklappenprothese und hält das Implantat (und insbesondere das selbstexpandierende Grundgerüst) im komprimierten Zustand. Fällt die Haltekraft der Implantatkapsel, beispielsweise durch eine axiale Verschiebung des Außenschaftes gegenüber dem Innenschaft, weg, expandiert das Grundgerüst aufgrund des Formgedächtniseffektes in seinen entfalteten Zustand. Die Herzklappenprothese (das Implantat) wird implantiert. Dies erfolgt zumeist über ein Zurückziehen des Außenschaftes (und damit der Implantatkapsel) nach proximal.

Mittels des Katheters wird die Herzklappenprothese in die richtige Position gebracht und nach Zurückziehen der Katheterhülse (in proximale Richtung) entfaltet. Hierbei muss die Katheterhülse jedoch in manchen Situationen auch zumindest teilweise wieder über die Herzklappenprothese geschoben werden (Resheathing, Wiedereinfangen), um eine Repositionierung der Herzklappenprothese zu ermöglichen.

Beim Zurückziehen der Katheterhülse wirken Biegekräfte direkt auf den Innenschaft, der dann nicht mehr von der Katheterhülse umgeben ist. Die Biegekräfte entstehen dabei automatisch, da sich der distale Bereich des Katheters beim Freisetzen der Herzklappenprothese im Aortenbogen befindet. Wenn ein Resheathing erforderlich ist, führen diese Biegekräfte auf den Innenschaft am distalen Ende der Katheterhülse zu einem Abwinkeln/Abknicken im Bereich des distalen Endes des Innenschafts, wodurch auch die Herzklappenprothese mechanisch stark beansprucht werden kann. Zudem wird hierdurch das Resheathing behindert.

Bekannte Systeme besitzen am distalen Ende der Katheterhülse Anordnungen, welche die Einführung der Herzklappenprothese während des Resheathing erleichtern bzw. sich konisch aufformen (flaring) und dadurch die Stützstruktur der Herzklappenprothese graduell umformen und führen, dass der Biegewinkel an der Katheterhülse und der Herzklappenprothese minimiert wird (self centering). Diese Lösungen zeichnen sich jedoch durch eine vergleichsweise große Dicke der Wand der Katheterhülse aus, so dass das für die Herzklappenprothese vorhandene Lumen begrenzt ist. Die Herzklappenprothese muss daher stärker gecrimpt werden und ist daher auch beim Wiedereinfangen einer höheren Belastung ausgesetzt hinsichtlich mechanischer Beanspruchung der Stützstruktur der Herzklappenprothese, des Materialgefüges und der Nähte der Herzklappenprothese. Zusätzlich besteht die Gefahr, dass Teile der Herzklappenprothese zwischen Struts der Stützstruktur eingeklemmt werden.

Die Aufgabe der vorliegenden Erfindung besteht somit darin, ein System aus einem Katheter und einem Implantat, insbesondere einer Herzklappenprothese zu schaffen, welche ein Abknicken während des Resheathing verhindert. Weiter besteht die Aufgabe darin, ein einfaches Verfahren zur Herstellung eines derartigen Systems anzugeben.

Die obige Aufgabe wird gelöst durch das System mit den Merkmalen des Anspruchs 1.

Das erfindungsgemäße System aus einem Katheter und einem Implantat weist insbesondere eine Herzklappenprothese mit einer selbstexpandierenden Stützstruktur auf. Der Katheter des erfindungsgemäßen Systems umfasst ferner einen Außenschaft und einen in dem Außenschaft angeordneten Innenschaft, wobei der Innenschaft einen röhrenförmigen Abschnitt aufweist, welcher eine Vielzahl von separaten, hohlzylinderförmigen, in Längsrichtung nebeneinander angeordneten und zueinander verschiebbaren sowie zueinander schwenkbaren Segmenten aufweist. Hierbei weist jedes Segment in Längsrichtung an einem Ende eine erste Anzahl n (n ≥ 2) sich jeweils in Längsrichtung (d.h. in distale oder proximale Richtung) erstreckende Zapfen (Nocken, Pins) und an dem anderen Ende in Längsrichtung jeweils eine der ersten Anzahl entsprechende Anzahl von Ausnehmungen mit einer zu der Form des jeweiligen Zapfens komplementären Form auf, wobei jeder Zapfen des unmittelbar benachbarten Segments in die jeweilige Ausnehmung mit der komplementären Form eingreift und umgekehrt, wobei eine zweite Anzahl m (m ≥ 1 und n ≥ m) der Zapfen des Segments eine den Abstand in Längsrichtung der benachbarten Segmente zueinander begrenzende Hinterschneidung aufweist.

Der röhrenförmige Abschnitt, welcher die Segmente aufweist, ist ungefähr so lang wie das Implantat, bevorzugt weist er mindestens 80% der Implantatlänge auf. Der röhrenförmige Abschnitt aufweisend die Segmente ist aber mindestens 20 mm, bevorzugt 30 mm, lang. Wenn der röhrenförmige Abschnitt deutlich kürzer als das Implantat ist, ist er zweckmäßigerweise mit einem Braid verstärkten Polymerschaft kombiniert.

Die Herzklappenprothese weist bevorzugt eine selbstexpandierende Stützstruktur auf, welche vorzugsweise im expandierten Zustand eine Stentform besitzt, aus einer Formgedächtnislegierung besteht und durch Überschreiten einer Umwandlungstemperatur oder Unterschreiten einer bestimmten Spannung von dem komprimierten in den expandierten Zustand übergeht. Die Expansion der Stützstruktur bewirkt dabei die Entfaltung der Herzklappenprothese. Innerhalb der Stützstruktur sind mindestens zwei, bevorzugt drei, aus einem biegsamen Material, bevorzugt Perikardgewebe, bestehende Klappensegel befestigt.

Bei dem erfindungsgemäßen System wird die Längsrichtung des Katheters beziehungsweise des Innenschafts beziehungsweise des röhrenförmigen Abschnitts als Richtung der Längsachse des Katheters beziehungsweise des Innenschafts beziehungsweise des röhrenförmigen Abschnitts verstanden. Die zueinander in Längsrichtung verschiebbaren Segmente sind nebeneinander in Reihe angeordnet und greifen mit ihren Zapfen und Ausnehmungen ineinander ein. Durch die Zapfen, welche eine Hinterschneidung (einen Hinterschnitt) aufweisen, wird der Abstand zweier benachbarter Segmente in Längsrichtung begrenzt. Nach der vorliegenden Erfindung sind die erste Anzahl n der Zapfen beziehungsweise Ausnehmungen und die zweite Anzahl m der Zapfen ganze Zahlen. Erfindungsgemäß weist ein Segment an einem Ende mindestens zwei sich in Längsrichtung erstreckende Zapfen auf (n ≥ 2). Vorzugsweise beträgt die Anzahl der Zapfen an einem Ende des Segments 4. Hierbei ist mindestens ein Zapfen als Zapfen mit Hinterschneidung ausgebildet (zweite Anzahl m, m ≥ 1). In einem Ausführungsbeispiel ist die Anzahl m der Zapfen mit Hinterschneidung an einem Ende kleiner als die Gesamtanzahl n der Zapfen an diesem Ende des Segments. Die Anzahl der Zapfen mit Hinterschneidung beträgt vorzugsweise 2. Dies bedeutet, dass an einem Ende des Segments Zapfen angeordnet sind, die keine Hinterschneidung aufweisen. In einem weiteren Ausführungsbeispiel weisen jedoch sämtliche Zapfen Hinterschneidungen auf. Hieraus ergibt sich n ≥ m.

Erfindungsgemäß haben Zapfen und Ausnehmungen benachbarter Segmente eine komplementäre Form, sodass ein Zapfen eines Segments in die entsprechende Ausnehmung mit der komplementären Form des benachbarten Segments eingreift. Hierbei sind Zapfen und Ausnehmung derart dimensioniert, dass ein gewisses Spiel, das heißt ein geringfügiger Abstand zwischen Zapfen und Ausnehmung, besteht, sodass benachbarte Segmente zueinander in Längsrichtung verschiebbar sind. Entsprechend sind benachbarte Segmente zueinander schwenkbar, wobei die Achse der Schwenkbewegung senkrecht zur Achse des Katheters (Längsrichtung) verläuft. Da Zapfen und Ausnehmungen benachbarter Segmente ineinander eingreifen, ist die Anzahl der Zapfen auf der einen Seite gleich der Anzahl der Ausnehmungen auf der anderen Seite, auch bezogen auf die jeweilige Form der Zapfen. Die Zapfen (und entsprechend die Ausnehmungen) eines Segments können sämtlich die gleiche Form oder unterschiedliche Formen aufweisen. Hierbei können Zapfen und Ausnehmungen, komplementärer oder verschiedener Form, an gegenüberliegenden Enden eines Segments auf einer Linie parallel zur Längsrichtung des röhrenförmigen Abschnitts liegen. Alternativ können sie auch entlang der Umfangsrichtung zueinander versetzt angeordnet sein.

Der vorzugsweise am distalen Ende des Innenschafts angeordnete röhrenförmige Abschnitt, der oben beschrieben wird, ermöglicht eine Auslenkung des Innenschafts bis zu einem bestimmten Grad, sodass der Innenschaft eine homogene Kurve ohne Diskontinuitäten beschreibt. Hierbei besitzt der Innenschaft nur einen kleinen Biegewiderstand, sodass das System insgesamt keine erhöhte Biegesteifigkeit besitzt, welche das Tracking erschweren würde. Mit dem erfindungsgemäßen System wird deshalb das Abknicken des Katheters beim Resheathing verhindert, sodass eine mechanische Beanspruchung der Herzklappenprothese verhindert wird. Hierdurch wird auch das Resheathing erleichtert.

In einem bevorzugten Ausführungsbeispiel weist jeder zweite oder dritte Zapfen entlang einer Umfangsrichtung eines Segments eine den Abstand der benachbarten Segmente begrenzende Hinterschneidung auf. Eine solche Konstruktion kann jedes Segment oder ein Teil der Segmente des röhrenförmigen Abschnitts betreffen. Die übrigen Zapfen weisen keine Hinterschneidung auf, sodass die Seitenflanken der Zapfen beispielsweise in Längsrichtung verlaufen und entlang ihrer gesamten Länge den gleichen Durchmesser aufweisen. Die Zapfen ohne Hinterschneidung sind beispielsweise fingerförmig ausgebildet. Hierdurch wird die Beweglichkeit der Segmente zueinander, insbesondere das Verschwenken benachbarter Segmente, weiter verbessert und der Krümmungsradius der Kurve, die der röhrenförmige Abschnitt bei dem Auftreten von Biegekräften beschreibt, wird verringert.

In einem weiteren Ausführungsbeispiel sind, in Längsrichtung betrachtet, die an dem einen Ende angeordneten Zapfen mit Hinterschneidung versetzt zu den an dem anderen Ende angeordneten Ausnehmungen mit komplementärer Form angeordnet. Der Versatz von Zapfen und komplementärer Ausnehmung erfolgt entlang der Umfangsrichtung.

Besonders effektive und einfach herzustellende Formen von Zapfen mit Hinterschneidungen sind erfindungsgemäß eine Schwalbenschwanzform oder eine Pilzform. Entsprechend weist mindestens die zweite Anzahl m der Zapfen eine Schwalbenschwanzform oder eine Pilzform auf.

Die Ausgestaltungen mit Zapfen in Schwalbenschwanzform oder Pilzform sind bevorzugt. Hierbei weist zweckmäßigerweise jedes Segment zwischen 3 und 15, bevorzugt zwischen 5 und 9, Zapfen auf.

Ebenfalls einfach zu fertigen ist ein System, bei dem in Längsrichtung an jedem Ende jedes Segments eine erste Anzahl von Zapfen und eine erste Anzahl von Ausnehmungen angeordnet sind, wobei ein Zapfen und eine Ausnehmung jeweils abwechselnd nebeneinander angeordnet sind. Hierbei bildet eine Seitenwand der Struktur an dem jeweiligen Ende des Segments sowohl eine Flanke eines Zapfens als auch eine Flanke einer Ausnehmung.

Die obige Aufgabenstellung wird außerdem durch ein röhrenförmiges Element für einen Innenschaft eines Katheters für ein System aus einem Katheter und einem Implantat, insbesondere einer Herzklappenprothese, mit einer selbstexpandierenden Stützstruktur gelöst, wobei das Element eine Vielzahl von separaten, hohlzylinderförmigen, in Längsrichtung nebeneinander angeordneten und zueinander verschiebbaren sowie zueinander schwenkbaren Segmenten aufweist, wobei jedes Segment in Längsrichtung an einem Ende eine erste Anzahl n (n ≥ 2) sich jeweils in Längsrichtung erstreckende Zapfen und an einem anderen Ende jeweils eine der ersten Anzahl entsprechender Anzahl von Ausnehmungen mit einer zu der Form des jeweiligen Zapfen komplementären Form aufweist, wobei jeder Zapfen des unmittelbar benachbarten Segments in die jeweilige Ausnehmung mit der komplementären Form eingreift und umgekehrt, wobei eine zweite Anzahl m (m ≥ 1 und n > m) der Zapfen des Segments eine den Abstand in Längsrichtung der benachbarten Segmente zueinander begrenzende Hinterschneidung aufweist. Ein Teil der Zapfen eines Segments weist erfindungsgemäß keine Hinterschneidung auf.

Ein derartiges erfindungsgemäßes Element weist eine besonders gute Beweglichkeit auf, wobei bei dem Einwirken von Biegekräften durch das röhrenförmige Element eine homogene, kontinuierliche Kurve beschrieben wird, die eine Abknicken eines Innenschafts während des Resheating verhindert.

Hierbei weist in einem Ausführungsbeispiel jeder zweite oder dritte Zapfen entlang einer Umfangsrichtung jedes Segments eine den Abstand der benachbarten Segmente begrenzende Hinterschneidung auf.

In einem weiteren bevorzugten Ausführungsbeispiel sind in Längsrichtung betrachtet die an dem einen Ende angeordneten Zapfen mit Hinterschneidung versetzt zu den an dem anderen Ende angeordneten Ausnehmungen mit komplementärer Form angeordnet.

Besonders einfach kann das oben beschriebene erfindungsgemäße System dadurch hergestellt werden, dass die einzelnen, separaten Segmente des röhrenförmigen Abschnitts aus einem Rohr, vorzugsweise mittels Laser, herausgeschnitten werden. Das Material des Innenschafts und entsprechend des röhrenförmigen Abschnitts kann beispielsweise einen Edelstahl enthalten. Alternativ kann der Innenschaft und entsprechend der röhrenförmige Abschnitt aus einem Kunststoff bestehen, insbesondere ein faserverstärkten Kunststoff. Ebenso sind Karbonfaser verstärkte Kunststoffe zweckmäßig. Für derartige faserverstärkte Kunststoffe sind dem Fachmann entsprechende Laserschneidverfahren oder auch Wasserschneidverfahren bekannt.

Weitere Ziele, Merkmale, Vorteile und Anwendungsmöglichkeiten der Erfindung ergeben sich auch aus der nachfolgenden Beschreibung von Ausführungsbeispielen der Erfindung anhand der Figuren. Dabei bilden alle beschriebenen und/oder bildlich dargestellten Merkmale für sich oder in beliebiger Kombination den Gegenstand der vorliegenden Erfindung, auch unabhängig von ihrer Zusammenfassung in den einzelnen Ansprüchen oder deren Rückbeziehung.

Es zeigen schematisch
- Fig. 1: einen Katheter eines erfindungsgemäßen Systems vor der Implantation einer Herzklappenprothese in einer perspektivischen Ansicht von der Seite,
- Fig. 2: einen distalen Abschnitt des Katheters aus Fig. 1 in einer perspektivischen Ansicht von der Seite nach dem Freisetzen der Herzklappenprothese,
- Fig. 3 und 4: weitere Ansichten eines distalen Abschnitts des Katheters aus Fig. 1 von der Seite nach dem Freisetzen der Herzklappenprothese und
- Fig. 5 bis 7: Ansichten von drei Ausführungsbeispielen eines Innenschafts des Katheters gemäß Fig. 1, jeweils von der Seite.

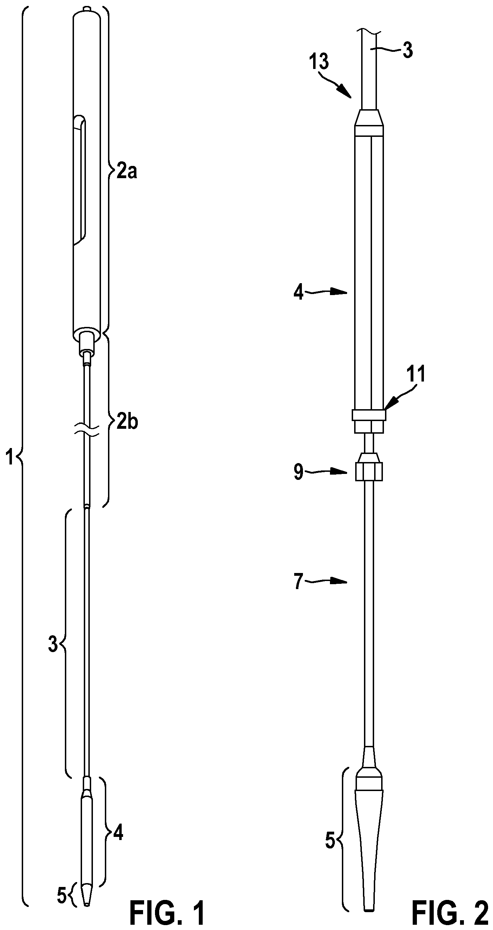

Fig. 1 zeigt einen Katheter 1 einer Ausgestaltung eines erfindungsgemäßen Systems aus Katheter und Herzklappenprothese mit einem am proximalen Ende des Katheters angeordneten Handgriff 2a, einen Stabilisatorabschnitt 2b, einem Außenschaft 3 sowie einer auf dem Außenschaft 3 angeordneten Katheterhülse 4. Am äußersten distalen Ende ist eine stumpfe Katheterspitze 5 vorgesehen. Der Stabilisatorabschnitt 2 (bestehend aus einem Stabilisatorschaft, der den Außenschaft umgibt) schirmt den zurückziehbaren Außenschaft 3 gegen ein Einführelement (Introducer) und die Gefäßwand ab, sodass der Außenschaft 3 frei zurückgezogen werden kann. Der Handgriff 2a dient dem Beladen, Freisetzen und Zurückziehen der in der Katheterhülse 4 angeordneten, nicht dargestellten selbstexpandierenden Herzklappenprothese. Eine derartige Herzklappenprothese weist im Wesentlichen einen selbstexpandierenden Stent, welcher die Stützstruktur des Implantats bildet, und eine darin befestigte Klappenanordnung auf, beispielsweise aus biologischem Gewebe (z.B. Perikardgewebe). Die Katheterspitze 5 bildet das distale Ende eines innerhalb des Außenschafts 3 angeordneten Innenschafts 7 (siehe Fig. 2), wobei die Katheterspitze 5 vorzugsweise aus PEBAX im Gemisch mit Bariumsulfat-Pulver bzw. Wolfram-Pulver gefertigt und unter Röntgenbestrahlung sichtbar ist.

Fig. 2 skizziert das distale Ende des in Fig. 1 gezeigten Systems nach dem Freisetzen der Herzklappenprothese. In dieser Figur ist auch zu sehen, dass auf dem Innenschaft 7 ein Prothesenkonnektor 9 angeordnet ist, mit dem die Herzklappenprothese auf dem Innenschaft 7 axial befestigt ist. Die Katheterhülse 4 kann in einem Ausführungsbeispiel an ihrem distalen Ende zur Erleichterung der Beobachtung einen unter Röntgenbestrahlung sichtbaren Ring 11 aufweisen. Die Katheterhülse 4 ist mittels eines proximalen Konnektors 13 mit dem Außenschaft 3 verbunden.

Wie bereits oben erläutert wurde, ist die Herzklappenprothese zunächst im komprimierten Zustand (siehe Fig. 1) in der Katheterhülse 4 (auch als Kapsel bezeichnet) angeordnet und wird durch die Katheterhülse 4 in diesem Zustand gehalten. Die Katheterhülse 4 ist über den Außenschaft 3 mit dem Handgriff 2a verbunden. In diesem Zustand erfolgt der Transport der in der Katheterhülse 4 fixierten und komprimierten Herzklappenprothese durch die Gefäße des Patienten an den Ort der Behandlung im Herzen.

Zum Freisetzen des Implantats wird die Katheterhülse 4 nach proximal zurückgezogen. Der Rückzug wird über den Handgriff 2a ausgelöst und über den Außenschaft 3 auf die Katheterhülse 4 übertragen. Hierbei wird zunächst lediglich ein kurzer distaler Abschnitt der Herzklappenprothese freigesetzt und der Sitz geprüft. Bei ungünstiger Positionierung wird die Katheterhülse mittels des Handgriffs 2a wieder nach distal zurückgeschoben (Resheathing), sodass die Herzklappenprothese wieder durch die Katheterhülse 4 bedeckt und vollständig in den komprimierten Zustand übergegangen ist. Nun wird der Katheter 1 repositioniert und das Freisetzen der in der Katheterhülse 4 angeordneten Herzklappenprothese beginnt von Neuem.

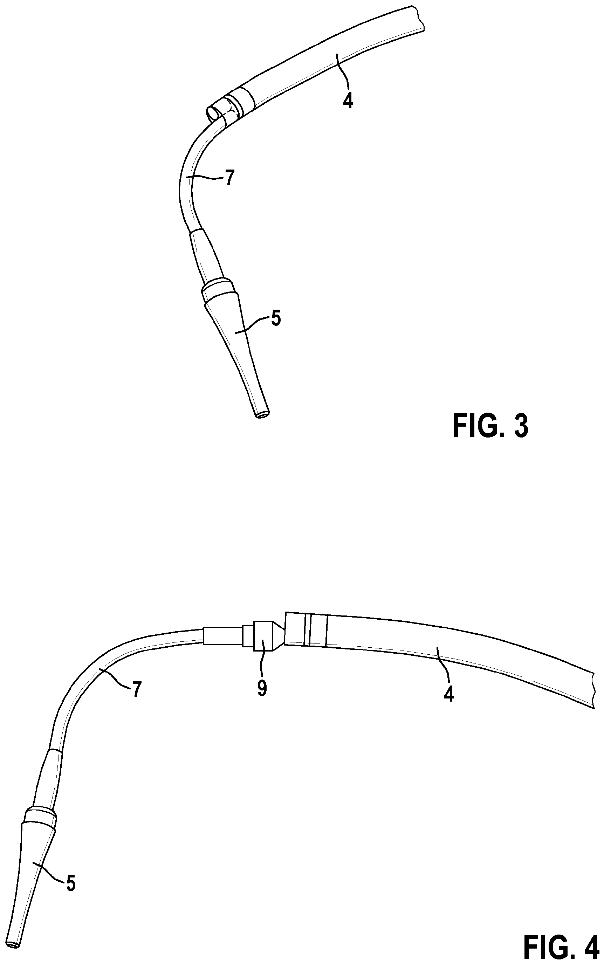

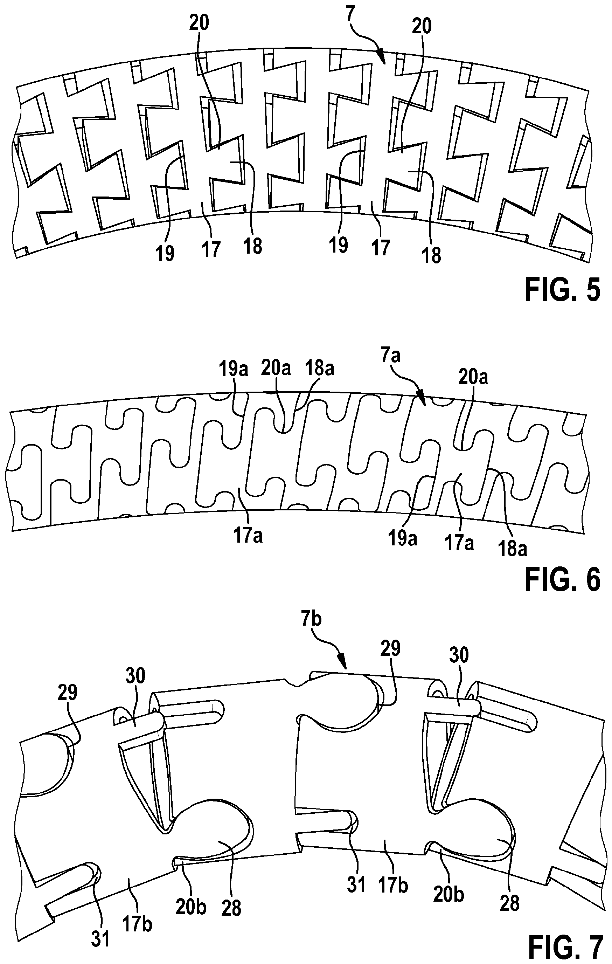

In den Figuren 3 und 4 wird das distale Ende des in Figur 1 skizzierten Systems nochmals detaillierter dargestellt. Insbesondere ist diesen Figuren zu entnehmen, dass der Innenschaft 7 mit einem distalen Abschnitt, der in den Figuren 5 bis 7 in verschiedenen Ausführungsbeispielen detaillierter dargestellt ist, bei dem Auftreten von Biegekräften eine homogene, kontinuierliche Kurve beschreibt, sodass ein Abknicken des Innenschafts 7 beim Resheathing verhindert wird.

Figur 5 zeigt ein erstes Ausführungsbeispiel eines distal angeordneten, röhrenförmigen Abschnitts eines Innenschafts 7. Der Innenschaft 7 weist eine Vielzahl von in Längsrichtung nebeneinander angeordneten, separaten Segmenten 17 auf. Die Segmente 17 sind in Längsrichtung zueinander verschiebbar und verschwenkbar. Jedes Segment 17 weist in Längsrichtung an dem einen Ende eine Anzahl 7 von schwalbenschwanzförmigen Zapfen 18 sowie an dem anderen Ende eine Anzahl 7 von zu der Schwalbenschwanz-Form des Zapfens 18 komplementären Ausnehmungen 19 auf. Der Zapfen 18 eines benachbarten Segments 17 ist jeweils in einer Ausnehmung 19 des Segments 17 angeordnet und umgekehrt. Die Verschiebbarkeit beziehungsweise Verschwenkbarkeit der Segmente 17 zueinander entsteht durch ein Spiel der jeweils ineinander angeordneten Ausnehmung 19 und Zapfen 18. Die Form des Zapfens 18 ist etwas kleiner als die Form der Ausnehmung 19, die durch ihre Innenwand gebildet wird. Durch eine Hinterschneidung 20 des schwalbenschwanzförmigen Zapfens 18 wird jedoch der Abstand bei einer Verschiebung der Segmente 17 in Längsrichtung zueinander und die Verschwenkbarkeit der Segmente 17 zueinander begrenzt.

Figur 6 zeigt ein weiteres Ausführungsbeispiel eines rohrförmigen Abschnitts eines Innenschafts 7a. Analog zu dem in Figur 5 gezeigten Ausführungsbeispiel weist jedes Segment 17a an dem einen Ende eine Anzahl von n Zapfen 18a und an dem anderen Ende eine Anzahl von n Ausnehmungen 19a auf, die in Bezug auf benachbarte Segmente 17a in einander angeordnet sind. Die Zapfen 18a und Ausnehmungen 19a besitzen bei diesem Ausführungsbeispiel eine Pilzform, sodass die Zapfen 18a eine Hinterschneidung 20a ausbilden. Das Spiel der ineinander greifenden Zapfen 18a und Ausnehmungen 19a ist bei dieser Ausführungsform geringer als bei der in Figur 5 gezeigten Ausführungsform, sodass die Verschwenkbarkeit und Verschiebbarkeit der Segmente 17a zueinander geringer ist und entsprechend die von dem Innenschaft 7a beschriebene Kurve einen größeren Radius aufweist. Bei diesem Ausführungsbeispiel sind ferner die Zapfen 18a an dem einen Ende eines Segments 17a versetzt zu den jeweils komplementären Ausnehmungen 19a angeordnet. Der Versatz erfolgte entlang der Umfangsrichtung des jeweiligen Segments 17a.

Das in Figur 7 gezeigte Ausführungsbeispiel eines röhrenförmigen Abschnitts eines Innenschafts 7b weist Segmente 17b auf, welche Zapfen und Ausnehmungen mit verschiedenen Formen besitzen. Jedes der nebeneinander in Längsrichtung angeordnetes Segment 17b besitzt in Längsrichtung an dem einen Ende eine Anzahl 2 erste Zapfen 28, welche eine Pilzform mit Hinterschneidung 20b aufweisen, und eine Anzahl 2 zweite Zapfen 30, die fingerförmig ausgebildet sind und keine Hinterschneidung ausbilden. Entsprechend weist jedes Segment 17b an dem anderen Ende mehrere erste Ausnehmungen 29, welche eine zu der Pilzform komplementäre Form besitzen, und mehrere zweite Ausnehmungen 31, die eine zu der Fingerform komplementäre Form zeigen. Der erste Zapfen 28 und der zweite Zapfen 30 beziehungsweise die erste Ausnehmung 29 und die zweite Ausnehmung 31 sind jeweils abwechselnd entlang der Umfangsrichtung an dem jeweiligen Ende des jeweilige Segments 17b angeordnet. Ferner sind in Längsrichtung gesehen der erste Zapfen 28 mit Hinterschneidung 20b und die erste dazu komplementär geformte Ausnehmung 29 versetzt angeordnet. Der Versatz erfolgt in Umfangsrichtung des jeweiligen Segments 17b. Entsprechend ist der zweite Zapfen 30 ohne Hinterschneidung in Längsrichtung gesehen versetzt zu der zweiten Ausnehmung 31 der komplementären Form angeordnet. Das in Figur 7 dargestellte Ausführungsbeispiel erlaubt aufgrund der Zapfen 30 ohne Hinterschneidung eine besonders gute Verschwenkbarkeit des Segmente 17b zueinander, da diese den Abstand benachbarter Segmente 17b an dieser Stelle nicht begrenzen. Hierdurch wird bei dem Einwirken von Biegekräften auf den Innenschaft 7b eine homogene, kontinuierliche Kurve ausgebildet, welche eine verglichen mit den in Figur 5 und 6 dargestellten Ausführungsbeispielen einen kleineren Radius aufweist.

### Bezugszeichenliste:

- 1: Katheter
- 2a: Handgriff
- 2b: Stabilisierabschnitt
- 3: Außenschaft
- 4: Katheterhülse
- 5: Katheterspitze
- 7, 7a, 7b: Innenschaft
- 9: Prothesenkonnektor
- 17, 17a, 17b: Segment
- 18, 18a: Zapfen
- 19, 19a: Ausnehmung
- 20, 20a, 20b: Hinterschneidung
- 28: erster Zapfen
- 29: erste Ausnehmung
- 30: zweiter Zapfen
- 31: zweite Ausnehmung

## Patentansprüche

1. System aus einem Katheter (1) und einem Implantat mit einer selbstexpandierenden Stützstruktur, wobei der Katheter einen Außenschaft (3) und einen in dem Außenschaft (3) angeordneten Innenschaft (7, 7a, 7b) umfasst, wobei der Innenschaft (7, 7a, 7b) einen röhrenförmigen Abschnitt aufweist, welcher eine Vielzahl von hohlzylinderförmigen, in Längsrichtung nebeneinander angeordneten und zueinander verschiebbaren sowie zueinander schwenkbaren Segmenten (17, 17a, 17b) aufweist, wobei jedes Segment (17, 17a, 17b) in Längsrichtung an einem Ende eine erste Anzahl n (n ≥ 2) sich jeweils in Längsrichtung erstreckende Zapfen (18, 18a, 28, 30) und an dem anderen Ende jeweils eine der ersten Anzahl entsprechende Anzahl von Ausnehmungen (19, 19a, 29, 31) mit einer zu der Form des jeweiligen Zapfens (18, 18a, 28, 30) komplementären Form aufweist, wobei jeder Zapfen (18, 18a, 28, 30) des unmittelbar benachbarten Segments in die jeweilige Ausnehmung (19, 19a, 29, 31) mit der komplementären Form eingreift und umgekehrt, wobei eine zweite Anzahl m (m ≥ 1 und n ≥ m) der Zapfen (18, 18a, 28) des Segments (17, 17a, 17b) eine den Abstand in Längsrichtung der benachbarten Segmente (17, 17a, 17b) zueinander begrenzende Hinterschneidung (20, 20a, 20b) aufweist.

2. System nach Anspruch 1, **dadurch gekennzeichnet, dass** der röhrenförmige Abschnitt am distalen Ende des Innenschafts (7, 7a, 7b) angeordnet ist.

3. System nach einem der vorhergehenden Ansprüche, **dadurch gekennzeichnet, dass** jeder zweite oder dritte Zapfen (28) entlang einer Umfangsrichtung eines Segments (17b) eine den Abstand der benachbarten Segmente (17b) begrenzende Hinterschneidung (20b) aufweist.

4. System nach einem der vorhergehenden Ansprüche, **dadurch gekennzeichnet, dass** die an dem einen Ende angeordneten Zapfen (18a, 28) mit Hinterschneidung (20a, 20b) eines Segments (17b) versetzt zu den an dem anderen Ende angeordneten Ausnehmungen (19a, 29) mit komplementärer Form angeordnet sind.

5. System nach einem der vorhergehenden Ansprüche, **dadurch gekennzeichnet, dass** mindestens die zweite Anzahl m der Zapfen (18, 18a, 28) eine Schwalbenschwanzform oder eine Pilzform aufweist.

6. System nach einem der vorhergehenden Ansprüche, **dadurch gekennzeichnet, dass** in Längsrichtung an jedem Ende jedes Segments (7, 7a, 7b) eine erste Anzahl von Zapfen (18, 18a) und eine erste Anzahl von Ausnehmungen (19, 19a) angeordnet ist, wobei ein Zapfen (18, 18a) und eine Ausnehmung (19, 19a) jeweils abwechselnd nebeneinander angeordnet sind.

7. Röhrenförmiges Element für einen Innenschaft (7, 7a, 7b) eines Katheters (1) für ein System aus einem Katheter (1) und einem Implantat mit einer selbstexpandierenden Stützstruktur, wobei das Element eine Vielzahl von hohlzylinderförmigen, in Längsrichtung nebeneinander angeordneten und zueinander verschiebbaren sowie zueinander schwenkbaren Segmenten (17, 17a, 17b) aufweist, wobei jedes Segment (7, 7a, 7b) in Längsrichtung an einem Ende eine erste Anzahl n (n ≥ 2) sich jeweils in Längsrichtung erstreckende Zapfen (18, 18a, 28, 30) und an dem anderen Ende jeweils eine der ersten Anzahl entsprechende Anzahl von Ausnehmungen (19, 19a, 29, 31) mit einer zu der Form des jeweiligen Zapfens (18, 18a, 28, 30) komplementären Form aufweist, wobei jeder Zapfen (18, 18a, 28, 30) des unmittelbar benachbarten Segments (17, 17a, 17b) in die jeweilige Ausnehmung (19, 19a, 29, 31) mit der komplementären Form eingreift und umgekehrt, wobei eine zweite Anzahl m (m ≥ 1 und n > m) der Zapfen (18, 18a, 28) des Segments (17, 17a, 17b) eine den Abstand in Längsrichtung der benachbarten Segmente zueinander begrenzende Hinterschneidung (20, 20a, 20b) aufweist.

8. Element nach Anspruch 7, **dadurch gekennzeichnet, dass** jeder zweite oder dritte Zapfen (28) entlang einer Umfangsrichtung jedes Segments (17b) eine den Abstand der benachbarten Segmente begrenzende Hinterschneidung (20b) aufweist.

9. Element nach einem der Ansprüche 7 oder 8, **dadurch gekennzeichnet, dass** in Längsrichtung betrachtet die an dem einen Ende eines Segments (17a, 17b) angeordneten Zapfen (18a, 28) mit Hinterschneidung (20a, 20b) versetzt zu den an dem anderen Ende angeordneten Ausnehmungen (19a, 29) mit komplementärer Form angeordnet sind.

10. Verfahren zur Herstellung eines Systems nach einem der Ansprüche 1 bis 6, **dadurch gekennzeichnet, dass** die einzelnen Segmente (17, 17a, 17b) aus einem Metallrohr vorzugsweise mittels Laser herausgeschnitten werden.
